# EUROPÄISCHE PATENTSCHRIFT

(11) **EP 1 711 080 B1**
(45) Veröffentlichungstag und Bekanntmachung des Hinweises auf die Patenterteilung: **16.01.2008**
(21) Anmeldenummer: 05707182.1
(22) Anmeldetag: 01.02.2005
(51) Int. Cl.: A43B 7/04, A43B 17/00

(54) **ELEKTRISCH BEHEIZBARE EINLEGESOHLE**
ELECTRICALLY HEATABLE INSOLE
SEMELLE CHAUFFANTE ELECTRIQUE

(30) Priorität: 02.02.2004 DE 102004006046
(43) Veröffentlichungstag der Anmeldung: 18.10.2006
(73) Patentinhaber: Therm-IC Products GmbH, 8200 Gleisdorf (AT)
(72) Erfinder: MACHER, David, A-8570 Voitsberg (AT); SCHREINER, Gerhard, A-8045 Graz (AT)
(74) Vertreter: Pfenning, Meinig & Partner GbR
(86) Internationale Anmeldenummer: PCT/EP2005/001100
(87) Internationale Veröffentlichungsnummer: WO 2005/072548

(56) Entgegenhaltungen:
- EP-A- 0 162 031
- DE-C1- 4 000 259
- DE-U1- 20 317 143
- US-A- 5 495 682
- US-B1- 6 657 164

## Beschreibung

Die Erfindung betrifft eine elektrisch beheizbare Einlegesohle nach dem Oberbegriff des Hauptanspruchs.

Aus der DE 39 04 603 A1 ist eine beheizbare Schuhsohle bekannt, die aus mehreren Schichten aufgebaut ist, wobei eine zur Wärmeerzeugung dienende Schicht von einer leitfähigen Kunststofffolie mit aufgetragenen Leiterbahnen gebildet ist. Im Mittelfußbereich der Sohle ist auf der Unterseite der Heizfolie ein Kaltleiter-Schaltelement vorgesehen, das die tatsächliche vorliegende Temperatur registriert und bei einer vorbestimmten Temperatur die Heizung einschaltet und bei Erreichen einer weiteren vorbestimmten Temperatur wieder ausschaltet. Im Bereich der Ferse ist eine aufladbare Batterie mit induktiver Aufladspule und Gleichrichter vorgesehen.

Aus der DE 4000259 C1 ist eine beheizbare Einlegesohle gemäss dem Oberbegriff des Anspruchs 1 bekannt.

Der Erfindung liegt die Aufgabe zugrunde, eine elektrisch beheizbare Einlegesohle zu schaffen, die flexible Einsatzmöglichkeiten vorsieht und einfach zu bedienen ist.

Die Aufgabe wird erfindungsgemäß durch die kennzeichnenden Merkmale des Hauptanspruchs in Verbindung mit den Merkmalen des Oberbegriffs gelöst.

Dadurch, dass die in der Sohle angeordnete Steuerschaltung eine Fernsteuervorrichtung zum Ein- und Ausschalten bzw. Steuern des Heizvorgangs aufweist, kann der Heizvorgang unabhängig von der erlangten Temperatur vom Benutzer in flexibler Weise eingesetzt und nach seinen Wünschen in einfacher Weise ein- und ausgeschaltet werden. Durch Vorsehen einer Schutzschaltung zum Abschalten der aufladbaren Batterie vom übrigen Stromkreis wird vermieden, dass bei Kurzschluss oder dergleichen eine zu große Hitzeentwicklung stattfindet, die gefährlich für den Benutzer werden kann.

Insbesondere kann die Heizelektrode durch pulsförmige Spannung angesteuert,werden. Die Frequenz dieser Pulse ist so abgestimmt, dass die Blutzirkulation zusätzlich angeregt wird. Da übliche Batterien bei tiefen Temperaturen an Leistung verlieren, besitzt diese Art des Heizvorgangs einen zweiten Vorteil: Die Batterien werden kurz belastet. Anschließend wird ihnen eine kurze Regenerierphase gegönnt, in der sich erholen können. Dies erhöht im hohen Maße die Leistungsfähigkeit bei tiefen Temperaturen sowie die Lebensdauer dieser Zellen.

Durch die in den Unteransprüchen angegebenen Maßnahmen sind vorteilhafte Weiterbildungen und Verbesserungen möglich.

Besonders vorteilhaft ist, dass die aufladbare Batterie ein Lithiumakkumulator ist, da die Akkus besonders flach sind und die Belastung durch den Fersendruck aushalten. Vorzugsweise können LI-Ionen- oder LI-Polymerakkus eingesetzt werden, da sie die notwendige Leistung bei kleiner, vor allem flacher Bauweise abgeben.

Der Auswahl der verwendeten Batterien kommt besondere Bedeutung zu: Einerseits müssen diese über eine entsprechend hohe Kapazität verfügen um annehmbare Heizzeiten und Temperaturen zu realisieren, andererseits müssen sie klein und leicht sein, um den Bewegungskomfort nicht einzuschränken. Dies erfordert eine hohe Energiedichte, wie sie beispielsweise mit herkömmlichen Nickelakkumulatoren nicht zu erreichen ist.

Wiederaufladbare Lithium Ionen Zellen besitzen eine geeignete hohe Energiedichte. Aufgrund der hohen Energiedichte stellen solche Batterien aber ein Gefahrenpotential dar. Ein Kurzschluss würde zu einer Explosion der Zellen führen. Des Weiteren müssen die Zellen auch vor Überströmen und vor Tiefentladung geschützt werden, beides Ereignisse, die zu einer vollständigen Zerstörung der Zellen führen. Erst das erfindungsgemäße Merkmal einer Schutzschaltung erlaubt die sinnvolle Verwendung solcher Zellen in einer Sohle.

Das schon mit normaler Luftfeuchtigkeit chemisch extrem reagierende Lithium muss auch gegen mechanische Beschädigung (Tritt auf Nagel) geschützt werden. Ein mechanischer Schutz wird durch einen Metallkäfig, der die Batterie umgibt, gewährleistet.

Alternativ kann auch Lithium-Ionen-Polymere als Batterien eingesetzt werden. Aufgrund ihrer Flexibilität sind diese für den Einsatz in einer Sohle besonders geeignet. Da es sich hier um trockene Batterien handelt, besteht keine Gefahr einer Explosion im Falle eines Kurzschlusses oder einer mechanischen Beschädigung (Tritt auf Nagel). Aber auch hier ist eine Schutzschaltung sinnvoll, da auch diese Art von Batterien vor Überlastung bzw. Überströmen und Tiefentladung geschützt werden müssen, um eine vollständige Zerstörung der Batterie zu verhindern.

Aufgrund seiner Eigenschaften wäre der Einsatz von Lithium-Ionen-Polymere dem Einsatz von oben beschriebenen Nasszellen vorzuziehen. Nachteilig aber ist, dass Lithium-Ionen-Polymere sehr teuer sind.

Vorzugsweise ist die aufladbare Batterie oder sind die aufladbaren Batterien im Fersenbereich und/oder im Fußmuldenbereich der Einlegesohle angeordnet.

Der Bereich zwischen Fußmulde und Zehen bleibt vorzugsweise ausgespart. Dies ist besonders vorteilhaft für die Abrollbewegung beim Gehen/Laufen. Diesbezüglich ist insbesondere der Einsatz von flexiblen Lithiumakkumulatoren zu betonen: Durch die Flexibilität ist der Tragekomfort der Einlegesohle erheblich verbessert. Als billigere Variante können auch Standardbatterien eingesetzt werden, um die zum Schutz förmlich ein starres Gehäuse gezogen ist.

Vorzugsweise weist die Heizelektrode Minimelf-Widerstände auf.

Minimelfs sind elektrische Miniaturwiderstände in zylindrischer Bauform, ca. 3mm lang und mit einem Durchmesser von ca. 1 mm. Diese Widerstände sind in der Sohle längs zu den auftretenden Biegelinien angeordnet.

Entscheidend ist hier die zylindrische Bauform und die spezielle Anordnung. Würden hier prismatische SMD-Widerstände eingesetzt werden, würden diese bei Biegebeanspruchung der Platine durch die rechteckige Auflagefläche sofort brechen.

Verbunden werden diese Widerstände über Leiterbahnen. Die Leiterbahnen sind flächenartig ausgeführt, um eine gute Wärmeableitung von der Heizelektrode an den Fuß zu erreichen. Des Weiteren ist die Heizelektrode in einen Körper aus Kunststoff eingebettet oder auf diesen angeordnet. Dadurch wird die benötigte Stabilität erreicht.

Vorzugsweise ist die Steuerschaltung eine Steuerschaltung zur stufenlosen Regelung der Stärke des Heizvorgangs.

Mit einem Stellschalter ist man auf eine geringe Anzahl von festen Einstellungen der Stärke des Heizvorgangs begrenzt, die Temperatur kann nur in groben Stufen verändert werden. Erfindungsgemäß sind aber in der Steuerschaltung elektronische Komponenten und Software vorgesehen. Dadurch kann die Höhe der Stufen gegenüber einem Stellschalter erheblich feiner aufgelöst werden. Die Auflösung ist so einstellbar, dass ein Träger der Sohle die Temperaturdifferenz zwischen benachbarten Temperaturstufen (fast) nicht mehr wahrnehmen kann. Es wird somit der Eindruck einer stufenlosen Regelung vermittelt.

Vorzugsweise weist die Fernsteuervorrichtung ein externes Bedienteil mit einem Betätigungselement, z.B. einem Druckknopf oder -taster auf, über den mittels eines Senders, z.B. eines IR-Senders oder eines Ultraschallsenders ein Ein- bzw. Aus-Signal an ein in der Sohle eingebautes Steuerteil der Fernsteuervorrichtung abgegeben wird, das wiederum die Heizung aktiviert bzw. deaktiviert.

In vorteilhafter Weise kann die Fernsteuervorrichtung auch einen berührungslosen Schalter aufweisen, wobei in dem Bedienteil ein Schaltelement und in dem Steuerteil ein zweites Schaltelement angeordnet sind.

In vorteilhafter Weise kann das Bedienteil (10) einen Empfänger aufweisen und die Fernsteuervorrichtung eine bidirektionale Fernsteuerung sein.

Bei einer Fernsteuerung muss damit gerechnet werden, dass die Signalübertragung teilweise gestört ist. Die dieser Weiterbildung zugrunde liegende Aufgabe ist somit, zu gewährleisten, dass Betriebszustand der jeweiligen Sohle mit den Einstellungen am Bedienteil oder mit der Anzeige am Bedienteil übereinstimmt. In anderen Worten: Fehler in der Signalübertragung sollen möglichst ohne Wirkung bleiben.

Die erfindungsgemäße Steuerung löst diese Aufgabe auf folgende Weise: Die Fernsteuervorrichtung und das Bedienteil sind beide mit Sende- und Empfangseinheiten ausgestattet. Die Fernsteuervorrichtung der jeweiligen Sohle kann mit dem Bedienteil kommunizieren. Über das Bedienteil stellt der Träger der Sohle den gewünschten Parameter, beispielsweise die Heizleistung, ein. Diese wird vom Bedienteil an die Fernsteuervorrichtung der jeweiligen Sohle weitergegeben.

Um zu gewährleisten, dass die Signale des Bedienteils die Sohle erreicht haben, d.h., dass beispielsweise die am Bedienteil gewählte Heizleistung auch tatsächlich von der Sohle eingestellt wird, ist eine Quittierung des Sendesignals vorgesehen: Die Fernsteuervorrichtung der jeweiligen Sohle bestätigt den Erhalt des Signals vom Bedienteil. Empfängt das Bedienteil von beiden Sohlen die Quittierung, so ist sichergestellt, dass in beiden Sohlen die gewählte Heizleistung anliegt.

Weitere Parameter, die auf diese Weise korrekt übermittelt werden können, sind Akkuspannung, Temperatur der jeweiligen Sohle und der Zustand der Heizelektrode bzw: des Heizelements, um einige Beispiele aufzuzählen.

Auch die Funktionstüchtigkeit des Bedienteils kann mit einer bidirektionalen Fernsteuerung überwacht werden: Fällt die Fernbedienung beispielsweise aus, so erkennt dies die Fernsteuervorrichtung darüber, dass ausgesendete Signale nicht mehr quittiert werden. Darauf hin kann entsprechende Reaktion folgen, wie beispielsweise ein Senken der Heizleistung.

Des Weiteren ist eine Identifikation der jeweiligen Empfangs- und Sendeeinheiten vorgesehen. Damit wird gewährleistet, dass für den Fall, dass sich mehrere Systeme auf engem Raum befinden, keine gegenseitige Störung dieser Systeme eintritt.

Besonders vorteilhaft ist, dass in dem Sohlengrundkörper ein Steckkontakt eingearbeitet ist, an dem ein Netzgerät zur Aufladung der Akkus anschließbar ist. Es kann aber auch eine berührungslose Aufladung der Akkus, z.B. durch Induktion, erfolgen.

Ein Ausführungsbeispiel der Erfindung ist in der Zeichnung dargestellt und wird in der nachfolgenden Beschreibung näher erläutert. Es zeigen:
- Fig. 1: eine Aussicht auf die erfindungsgemäße Einlegesohle, wobei Teile der Abdeckungsschicht ausgeschnitten sind,
- Fig. 2: eine schematische Ansicht auf das Bedienteil der Fernsteuerung,
- Fig. 3: eine Seitenansicht auf den hinteren Teil einer Einlegesohle, und
- Fig. 4: eine schematische Ansicht auf eine Heizelektrode.

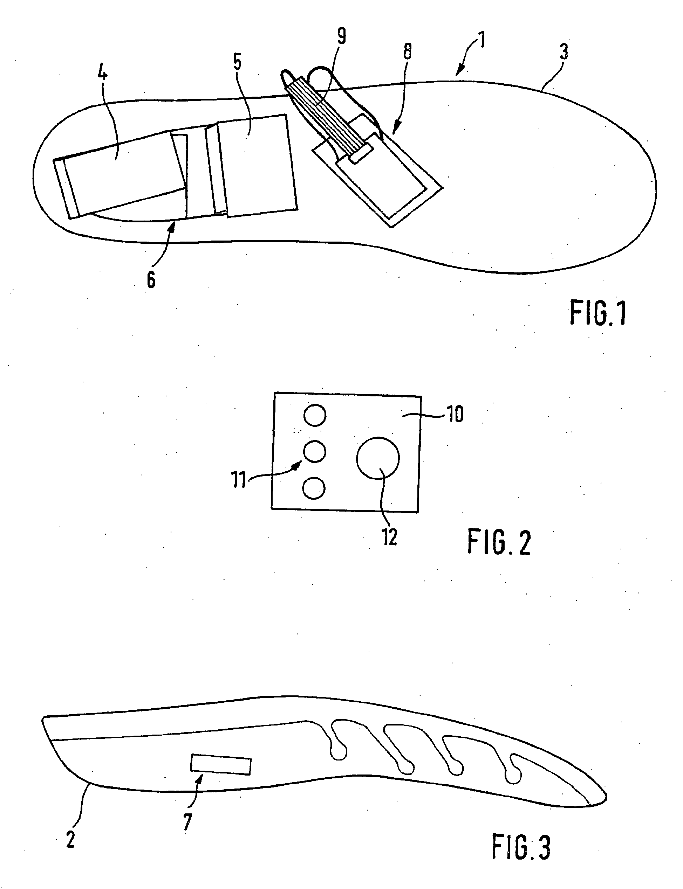

Die in Fig. 1 und Fig. 3 dargestellte Einlegesohle umfasst einen Sohlengrundkörper 2, der beispielsweise einer orthopädischen Sporteinlage entspricht und aus einem Formkörper oder aus mehreren Schichten bestehen kann, und eine Abdeckschicht 3, die dem Fuß des Benutzers zugewandt ist.

In den Sohlengrundkörper 2 sind elektronische Bauelemente eingebettet, wobei im Fersenbereich Vertiefungen für zwei Lithiumakkus 4, 5 vorgesehen sind, die als LI-Ionen- oder LI-Polymerakkuzellen ausgebildet sein können. Weiterhin ist eine vorzugsweise verkapselte Schutz- und Ladeschaltung 6 in den Grundkörper 2 eingesetzt, wobei die Schutzschaltung bei großer Hitzeentwicklung die Akkus von der übrigen Schaltung trennt. Nicht in der Figur zu sehen sind die Heiz-elektroden, die insbesondere im vorderen Fußbereich unter der Abdeckschicht 3 großflächig angeordnet sind und die aus Widerstandsflächen bestehen. Die Heizelektroden sind über die Schutzschaltung 6 mit den Akkus 4, 5 verbunden. Die Schutzschaltung weist Temperatur- und Stromsensoren und eine intelligente Logik auf, die die Steuerung bzw. Abschaltung der Heizelektroden übernimmt. Zusätzlich sind die Lithiumakkus in ein Schutzgehäuse eingebaut, damit der Benutzer im Fall einer Explosion der Batterie keinen Schaden erleidet. Es wird in diesem Fall die Druckwelle absorbiert und die Hitze in dem Schuh nach unten abgeleitet.

In dem dargestellten Ausführungsbeispiel ist die Ladeschaltung 6 mit einem Steckkontakt 7 verbunden, der gleichfalls in dem Sohlengrundkörper 2 eingebaut ist und seine Steckelemente nach außen freigibt. An diesen Steckkontakt kann ein Netzgerät zum Laden angeschlossen werden.

Weiterhin ist in den Grundkörper 2 eine Fernsteuerung 8. in Form einer bestückten Leiterplatte eingesetzt, die mit der Schutz- und Ladeschaltung 6 und den nicht dargestellten Heizungselektroden verbunden ist, und die die Ein- und Abschaltung der Heizelektroden steuert. Die Anschlussleitungen 9 sind hier oberhalb der Abdeckschicht 3 gezeigt. Selbstverständlich sind sie gleichfalls unterhalb der Abdeckschicht in dem Sohlengrundkörper eingesetzt. Die Fernsteuerung weist üblicherweise eine Sende- und Empfangseinrichtung auf, die mit Infrarotstrahlen oder Ultraschall oder sonstigen modulierten Funksignalen arbeitet. Die Frequenzbereiche können im Langwellen-, Mikrowellen-, HF- oder UHF-Bereich liegen.

Weiterhin sind elektronische Schaltelemente vorgesehen, die die Spannungsversorgung zwischen Akkus 4, 5 und Heizelementen steuern. Außerdem können Temperatursensoren vorgesehen sein, die gleichfalls zur Steuerung der Heizelemente dienen.

Das Bedienteil 10 für die Fernsteuerung, das beispielsweise als Schlüsselanhänger oder dergleichen ausgebildet sein kann und schematisch in Fig. 2 dargestellt ist, weist gleichfalls eine Sende- und Empfangsvorrichtung auf, die Signale zu dem Fernsteuerteil 8 in der Sohle liefert und von diesem empfängt. Weiterhin sind mehrere Anzeigeelemente 11 vorgesehen, die als LEDs ausgebildet sein können, und die den Einschaltzustand und/oder Temperaturen anzeigen. Weiterhin weist das Bedienteil 10 einen Druckknopf oder Drucktaster 12 auf, der ein Einschalt- bzw. Ausschaltsignal oder Wählsignale für die Temperaturstufen erzeugt, das über die Sende- und Empfangseinrichtung zu dem Fernsteuerteil 8 in der Sohle 1 übertragen wird.

Des Weiteren ist die Steuerschaltung als "stufenlose" Regelung ausgebildet. Notwendige Elektronik und Software sind in der Steuerschaltung integriert. Der Träger kann über das Bedienteil 10 die für ihn angenehmste Temperatur einstellen. Die Einstellung wird über den Drucktaster 12 getätigt.

Die Steuerschaltung ist eine Steuerschaltung für einen pulsförmigen Heizvorgang, d.h., es werden Heizpulse mit einer auf die Blutzirkulation abgestimmten Frequenz ausgesendet. Alternativ kann auch ein konstanter Heizvorgang vorgesehen sein.

Die Fernsteuerung dieses Systems Sohlen-Bedienteil ist bidirektional ausgestaltet. Die jeweiligen empfangenen Signale werden über ein Antwortsignal quittiert. Damit ist gewährleistet, dass der Empfänger das Signal erhalten hat, dass beispielsweise die am Bedienteil 10 gewählte Temperatur auch tatsächlich an die Fernsteuervorrichtung in den Sohlen weitergegeben wurde.

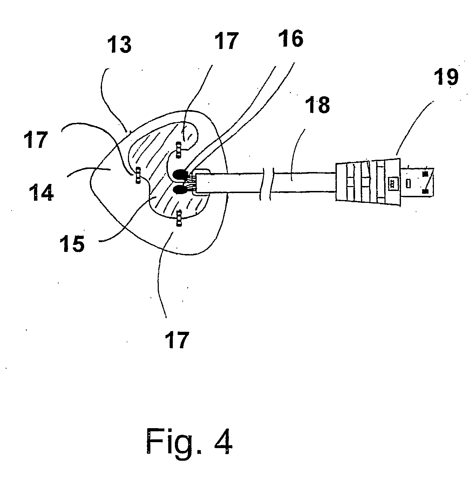

Die Figur 4 zeigt eine schematische Ansicht einer erfindungsgemäßen Heizelektrode.

Gezeigt ist eine Platine 13. Die Platine ist im vorderen Bereich in der Sohle eingebettet. Sie deckt diesen vorderen Bereich großflächig ab. Die Platine besteht aus Epoxid-Harz und ist flexibel. Dadurch bleibt auch die Flexibilität der Sohle erhalten. Alternativ sind aber auch andere flexible Kunststoffe bzw. Kunstharze einsetzbar.

Die Platine ist nahezu vollständig mit Kupfer beschichtet. Dabei ist diese Kupferschicht galvanisch in einen ersten Bereich 14 und in einen zweiten Bereich 15 getrennt. Diese beiden Bereiche werden mit zwei Elektroden 16 kontaktiert. Über Kabel 18 und einen Stecker 19 sind die Elektroden 16 mit der Steuerschaltung, in dieser Figur nicht dargestellt, verbunden.

Die elektrische Verbindung zwischen den ersten Bereich 14 und den zweiten Bereich 15 wird über drei parallel geschaltete zylindrische Minimelf-Widerstände 17 mit einem Widerstand von 27 Ohm hergestellt. Diese Widerstände sind direkt auf die Kupferschicht gelötet. Sie sind in Längsrichtung nach den Biegelinien der Sohle ausgerichtet.

Die Widerstände sind so auf der Platine angeordnet, dass sie jeweils einen möglichst großen Bereich der Platine abdecken, der sich mit dem Bereich eines anderen Widerstands nicht überschneidet.

Alternativ kann auch eine andere Anzahl von Widerständen eingesetzt werden. Dies ist unter anderem abhängig von der Größe der Fläche, die man Erwärmen möchte.

Wird nun an die Elektroden 16 eine Spannung angelegt, so erhitzen sich die Widerstände 17. Da diese Widerstände direkt auf die Bereiche 14 und 15 der Kupferschicht gelötet sind, geben diese Widerstände ihre Wärme unmittelbar an die Kupferschicht ab. Durch die Anordnung der Widerstände werden große Wärmeverteilungen oder Temperaturgradienten auf der Kupferschicht verhindert. Die Kupferschicht verteilt die Wärme schnell und großflächig.

In einem anderen Ausführungsbeispiel kann zum Ein-und Ausschalten ein berührungsloser Schalter verwendet werden, der beispielsweise als Magnetschalter, Näherungsschalter oder dergleichen ausgebildet ist und bei dem ein erstes Schaltelement anstelle des Druckknopfes 12 in dem Bedienteil 10 vorgesehen ist und ein zweites Schaltelement in der so genannten "Fernsteuerung" in der Sohle angeordnet ist. In diesem Fall kann gleichfalls in der Sohle ein Sender vorgesehen sein und im Bedienteil 10 ein Empfänger, um Informationen über den Einschaltzustand und gegebenenfalls Temperaturen an die Anzeigeelemente 11 zu senden.

In noch einem anderen Ausführungsbeispiel können diese Anzeigeelemente weggelassen werden und das Bedienteil 10 besteht lediglich aus dem ersten Schaltelement.

## Patentansprüche

1. Elektrisch beheizbare Einlegesohle mit mindestens einem Sohlengrundkörper und einer Abdeckschicht, mindestens einer Heizelektrode, mindestens einer mit der Heizelektrode elektrisch verbundenen aufladbaren Batterie sowie einer Steuerschaltung zum Steuern des Heizvorgangs und des Aufladens der Batterie, wobei Heizelektrode, aufladbare Batterie und Steuerschaltung in dem Sohlengrundkörper und/oder zwischen Sohlengrundkörper und Abdeckschicht angeordnet sind, **dadurch gekennzeichnet, dass** die Steuerschaltung (6, 8) eine Fernsteuervorrichtung zum Ein- und Ausschalten des Heizvorgangs und eine Schutzschaltung zum Abschalten der aufladbaren Batterie im Fehlerfalls aufweist.

2. Einlegesohle nach Anspruch 1, **dadurch gekennzeichnet, dass** die mindestens eine aufladbare Batterie (4, 5) ein Lithiumakkumulator ist.

3. Einlegesohle nach einen der Ansprüche 1 bis 2, **dadurch gekennzeichnet, dass** die aufladbare Batterie oder die aufladbaren Batterien im Fersenbereich und/oder im Fußmuldenbereich der Einlegesohle angeordnet sind.

4. Einlegesohle nach einen der Ansprüche 1 bis 3, **dadurch gekennzeichnet, dass** die Heizelektrode Minimelf-Widerstände aufweist.

5. Einlegesohle nach einen der Ansprüche 1 bis 4, **dadurch gekennzeichnet, dass** die Steuerschaltung eine Steuerschaltung zur stufenlosen Regelung der Stärke des Heizvorgangs ist.

6. Einlegesohle nach Anspruch 1 bis 5, **dadurch gekennzeichnet, dass** die Fernsteuervorrichtung (8) ein externes Bedienteil (10) und ein eingebautes Steuerteil aufweist.

7. Einlegesohle nach einem der Ansprüche 1 bis 6, **dadurch gekennzeichnet, dass** die Fernsteuervorrichtung einen berührungslosen Schalter aufweist.

8. Einlegesohle nach einem der Ansprüche 1 bis 7, **dadurch gekennzeichnet, dass** die Fernsteuervorrichtung eine Sende- und Empfangseinheit zur drahtlosen Übertragung von Informationen aufweist.

9. Einlegesohle nach einem der Ansprüche 5 bis 8, **dadurch gekennzeichnet, dass** das Bedienteil (10) Anzeigeelemente (11) zur Funktionsanzeige und/oder Temperaturanzeige aufweist.

10. Einlegesohle nach einem der Ansprüche 5 bis 9, **dadurch gekennzeichnet, dass** das Bedienteil (10) einen Sender und ein Betätigungselement (12) zum Aktivieren des Senders aufweist, der im aktivierten Zustand ein drahtloses Einschaltsignal an das eingebaute Steuerteil sendet.

11. Einlegesohle nach einem der Ansprüche 8 bis 10, **dadurch gekennzeichnet, dass** das Bedienteil (10) einen Empfänger aufweist und dass die Fernsteuervorrichtung eine bidirektionale Fernsteuerung ist.

12. Einlegesohle nach einem der Ansprüche 5 bis 11, **dadurch gekennzeichnet, dass** das Bedienteil (10) ein erstes Schaltelement eines berührungslosen Schalters aufweist, das mit einem zweiten, im eingebauten Steuerteil angeordneten Schaltelement zusammenarbeitet.

13. Einlegesohle nach einem der Ansprüche 5 bis 12, **dadurch gekennzeichnet, dass** der berührungslose Schalter als Reed-Schalter, Magnetschalter, Näherungsschalter oder dergleichen ausgebildet ist.

14. Einlegesohle nach einem der Ansprüche 5 bis 13, **dadurch gekennzeichnet, dass** in den Sohlengrundkörper (2) ein mit der Steuerschaltung (6, 8) verbundener Steckkontakt für den Anschluss eines Netzgerätes eingebaut ist.

## Claims

1. An electrically heatable insole with at least one basic sole body and one covering layer, at least one heating electrode, at least one rechargeable battery electrically connected to the heating electrode, and a control circuit for controlling the heating process and the charging of the battery, wherein the heating electrode, rechargeable battery and control circuit are arranged in the basic sole body and/or between the basic sole body and the covering layer, **characterised in that** the control circuit (6, 8) has a remote-control device for starting and stopping the heating process and a protective circuit for disconnecting the rechargeable battery in the event of a fault.

2. An insole according to claim 1, **characterised in that** the at least one rechargeable battery (4, 5) is a lithium storage battery.

3. An insole according to either one of claims 1 to 2, **characterised in that** the rechargeable battery or the rechargeable batteries are arranged in the heel region and/or in the instep region of the insole.

4. An insole according to any one of claims 1 to 3, **characterised in that** the heating electrode has minimelf resistors.

5. An insole according to any one of claims 1 to 4, **characterised in that** the control circuit is a control circuit for continuously adjusting the intensity of the heating process.

6. An insole according to claims 1 to 5, **characterised in that** the remote-control device (8) has an external operating part (10) and a built-in control part.

7. An insole according to any one of claims 1 to 6, **characterised in that** the remote-control device has a contactless switch.

8. An insole according to any one of claims 1 to 7, **characterised in that** the remote-control device has a transmitting and receiving unit for the wireless transmission of information.

9. An insole according to any one of claims 5 to 8, **characterised in that** the operating part (10) has indicating elements for functional indication and/or temperature indication.

10. An insole according to any one of claims 5 to 9, **characterised in that** the operating part (10) has a transmitter and an actuating member (12) for activating the transmitter which, in the activated state, transmits a wireless start signal to the built-in control part.

11. An insole according to any one of claims 8 to 10, **characterised in that** the operating part (10) has a receiver and **in that** the remote-control device is a bidirectional remote control.

12. An insole according to any one of claims 5 to 11, **characterised in that** the operating part (10) has a first switching element of a contactless switch, which first switching element co-operates with a second switching element arranged in the built-in control part.

13. An insole according to any one of claims 5 to 12, **characterised in that** the contactless switch is formed as a reed switch, magnetic switch, proximity switch or the like.

14. An insole according to any one of claims 5 to 13, **characterised in that** a plug contact for the connection of a power pack is connected to the control circuit (6, 8) and is built into the basic sole body (2).

## Revendications

1. Semelle chauffante électrique comportant au moins un fond de semelle et une couche de recouvrement, au moins une électrode de chauffage, au moins une batterie rechargeable reliée électriquement à l'électrode chauffante ainsi qu'un circuit de commande pour la commande du processus de chauffage ainsi que du chargement de la batterie, l'électrode de chauffage, la batterie rechargeable et le circuit de commande étant disposés dans le fond de la semelle et/ou entre le fond de la semelle et la couche de recouvrement,
**caractérisée en ce que**
le circuit de commande (6, 8) comporte un dispositif de télécommande pour la mise en marche et l'arrêt du processus de chauffage et un circuit de protection pour la coupure de la batterie rechargeable en cas de défaut.

2. Semelle selon la revendication 1, **caractérisée en ce que** la au moins une batterie rechargeable (4, 5) est un accumulateur au lithium.

3. Semelle selon l'une quelconque des revendications 1 à 2, **caractérisée en ce que** la batterie rechargeable ou les batteries rechargeables sont montées dans la zone du talon et/ou dans la zone de la voûte plantaire.

4. Semelle selon l'une quelconque des revendications 1 à 3, **caractérisée en ce que** l'électrode de chauffage présente des résistances Minimelf.

5. Semelle selon l'une quelconque des revendications 1 à 4, **caractérisée en ce que** le circuit de commande est un circuit de commande pour la régulation linéaire de la puissance du processus de chauffage.

6. Semelle selon l'une quelconque des revendications 1 à 5, **caractérisée en ce que** le dispositif de télécommande (8) comprend un organe de manoeuvre externe (10) et une partie commande intégrée.

7. Semelle selon l'une quelconque des revendications 1 à 6, **caractérisée en ce que** le dispositif de télécommande comporte un interrupteur sans contact.

8. Semelle selon l'une quelconque des revendications 1 à 7, **caractérisée en ce que** le dispositif de télécommande comporte une unité d'émission et de réception pour la transmission sans fil d'informations.

9. Semelle selon l'une quelconque des revendications 5 à 8, **caractérisée en ce que** l'organe de manoeuvre (10) présente des éléments d'affichage (11) pour la visualisation de fonctions et/ou la visualisation de la température.

10. Semelle selon l'une quelconque des revendications 5 à 9, **caractérisée en ce que** l'organe de manoeuvre (10) comporte un émetteur et un élément d'actionnement (12) pour l'activation de l'émetteur qui à l'état activé, envoie un signal de mise en marche sans fil à la partie commande intégrée.

11. Semelle selon l'une quelconque des revendications 8 à 10, **caractérisée en ce que** l'organe de manoeuvre (10) comporte un récepteur et **en ce que** le dispositif de télécommande est une télécommande bidirectionnelle.

12. Semelle selon l'une quelconque des revendications 5 à 11, **caractérisée en ce que** l'organe de manoeuvre (10) comporte un premier élément de commutation d'un interrupteur sans contact qui coopère avec un second élément de commutation disposé dans la partie commande intégrée.

13. Semelle selon l'une quelconque des revendications 5 à 12, **caractérisée en ce que** l'interrupteur sans fil est conçu sous forme d'interrupteur Reed, d'interrupteur magnétique, de détecteur de proximité ou similaire.

14. Semelle selon l'une quelconque des revendications 5 à 13, **caractérisée en ce qu'**un contact enfichable relié au circuit de commande (6, 8) est intégré dans le fond de semelle (2) pour le raccordement d'un bloc d'alimentation.
